# EUROPEAN PATENT SPECIFICATION

(11) **EP 1 562 506 B1**
(45) Date of publication and mention of the grant of the patent: **13.05.2009**
(21) Application number: 03783506.3
(22) Date of filing: 17.11.2003
(51) Int. Cl.: A61B 18/14

(54) **ELECTROPHYSIOLOGY CATHETER WITH ABLATION ELECTRODE**
ELEKTROPHYSIOLOGIEKATHETER MIT ABLATIONSELEKTRODE
CATHETER D'ELECTROPHYSIOLOGIE A ELECTRODE D'ABLATION

(30) Priority: 15.11.2002 US 426735 P
(43) Date of publication of application: 17.08.2005
(73) Proprietor: C.R.Bard, Inc., Murray Hill, NJ 07974 (US)
(72) Inventor: KOZEL, Peter, D., Concord, MA 01742 (US)
(74) Representative: Driver, Virginia Rozanne
(86) International application number: PCT/US2003/036488
(87) International publication number: WO 2004/045442

(56) References cited:
- EP-A- 1 151 726
- US-A- 6 030 382
- US-A1- 2002 072 744
- US-B1- 6 280 441
- US-B1- 6 287 306
- US-B1- 6 413 255
- US-B1- 6 456 863

## Description

### Background

The human heart is a very complex organ, which relies on both muscle contraction and electrical impulses to function properly. The electrical impulses travel through the heart walls, first through the atria and then the ventricles, causing the corresponding muscle tissue in the atria and ventricles to contract. Thus, the atria contract first, followed by the ventricles. This order is essential for proper functioning of the heart.

In some individuals, the electrical impulses of the heart develop an irregular propagation, disrupting the heart's normal pumping action. The abnormal heartbeat rhythm is termed a "cardiac arrhythmia." Arrhythmias may occur when a site other than the sinoatrial node of the heart is initiating rhythms (i.e., a focal arrhythmia), or when electrical signals of the heart circulate repetitively in a closed circuit (i.e., a reentrant arrhythmia).

Techniques have been developed which are used to locate cardiac regions responsible for the cardiac arrhythmia, and also to disable the short-circuit function of these areas. According to these techniques, electrical energy is applied to a portion of the heart tissue to ablate that tissue and produce scars which interrupt the reentrant conduction pathways or terminate the focal initiation. The regions to be ablated are usually first determined by endocardial mapping techniques. Mapping typically involves percutaneously introducing a catheter having one or more electrodes into the patient, passing the catheter through a blood vessel (e.g. the femoral vein or artery) and into an endocardial site (e.g., the atrium or ventricle of the heart), and deliberately inducing an arrhythmia so that a continuous, simultaneous recording can be made with a multichannel recorder at each of several different endocardial positions. When an arrythormogenic focus or inappropriate circuit is located, as indicated in the electrocardiogram recording, it is marked by various imaging or localization means so that cardiac arrhythmias emanating from that region can be blocked by ablating tissue. An ablation catheter with one or more electrodes can then transmit electrical energy to the tissue adjacent the electrode to create a lesion in the tissue. One or more suitably positioned lesions will typically create a region of necrotic tissue which serves to disable the propagation of the errant impulse caused by the arrythromogenic focus. Ablation is carried out by applying energy to the catheter electrodes. The ablation energy can be, for example, RF, DC, ultrasound, microwave, or laser radiation.

It is known that, rather than using a cylindrical or ring-shaped electrode, and electrode may be formed by wrapping a conductor successively around a catheter so that adjacent "windings" of the conductor touch each other. Such a configuration is generally employed to simulate the electrical behaviour of a ring-shaped electrode but at the same time to make the electrode covered portion of the catheter flexible, thereby permitting the electrode to form curved lesion patterns.

It is also known that certain physical and electrical advantages can be achieved by introducing spaces between the successive windings of the electrodes. Such advantages are discussed, for example, in US Patent No. 6,030,382 ("the '382 patent"). The electrode configurations described in the '382 patent, however, still suffer from a number of significant drawbacks that limit their performance capabilities. Those disadvantages, and the manner in which various aspects of the invention can be employed to overcome them, are discussed below.

Reference is also made to US 6,287,306 which describes a tissue ablating electrode. US 6,287,306 discusses that the so-called "edge effects" of an electrode can be ameliorated by altering the resistance profile along the electrode's length. In particular, for a cylindrically shaped electrode, the use of a higher resistance material at the end than is used in the middle can lower current densities in the ends relative to those in the middle, thus reducing edge effects.

There is no discussion in this reference about how to otherwise address the issue of edge effects.

According to one aspect of the present invention there is provided an apparatus for ablating tissue, comprising: a shaft; and a tissue-ablating electrode comprising a first end portion and a middle portion supported by respective lengthwise sections of the shaft, **characterised in that** the total energy-emitting surface area of the electrode per unit length of the shaft is greater for the middle portion of the electrode than for the first end portion of the electrode.

In one embodiment, a shaft-mounted electrode for ablating tissue comprises at least two separate coiled conductors having interleaved spirals.

In another embodiment, a shaft-mounted electrode for ablating tissue comprises a coiled conductor having spaces between at least some of its spirals. The electrode is mounted on the shaft such that at least a portion of an end of the electrode is disposed at least partially below an annular surface of the shaft that is adjacent the end of the electrode.

In another embodiment, a shaft-mounted electrode for ablating tissue comprises an end portion and a middle portion, and has at least one energy emitting area configured in a shape other than a coil. The electrode further comprises means for introducing edge effects in at least the middle portion.

### Brief Description of the Drawings

Figure 1 is a block diagram illustrating a system in which embodiments of the present invention may be employed; and
Figs. 2-11 each shows a distal end of a catheter having one or more electrodes mounted on it in accordance with a respective embodiment of the invention.

### Detailed Description

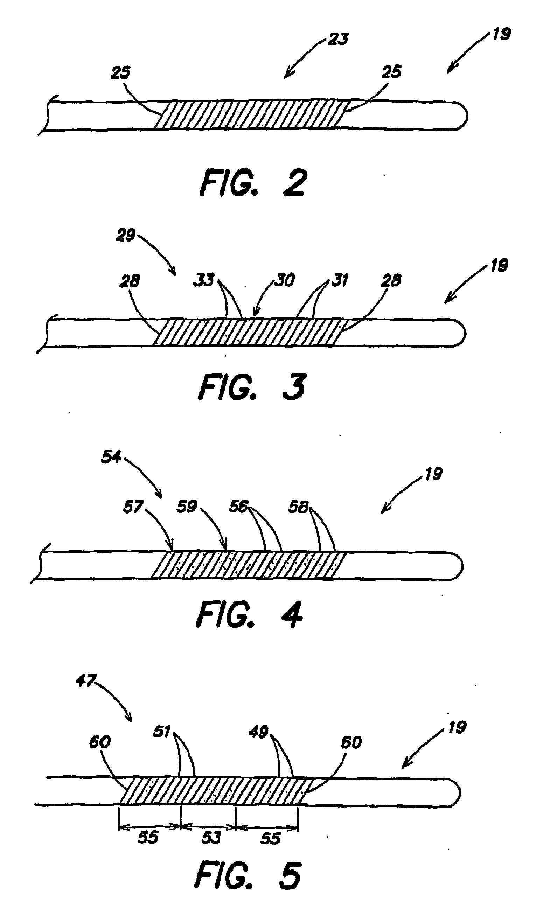

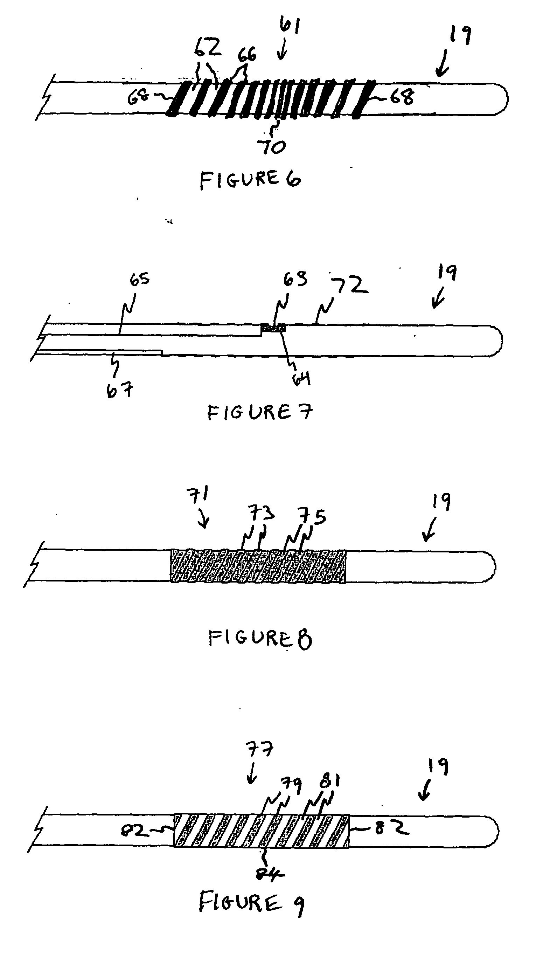

A conventional ring-shaped electrode exhibits a non-uniform electric field when energized. In particular, the electric field will be strongest at the ends of the electrode due to the increased current density caused by so-called "edge effects" (also sometimes referred to as "fringing" or "charge crowding") in those regions. Coiled electrodes that have abutting windings, like those depicted in Figs. 5 and 6 of the '382 patent, behave similarly to conventional electrodes in terms of their exhibited edge effects.

Recognizing the foregoing, two alternative approaches are disclosed herein for ameliorating this undesirable result. The resulting structures are electrodes that can create at least some basic types of lesion patters as conventional electrodes, but that emit ablation energy of a more uniform density along the electrode's entire length, thereby creating more uniform lesion patterns than their predecessors.

The first such approach disclosed herein involves introducing additional edge effects throughout the electrode to thereby minimize the adverse impact of the edge effects at the ends. That is, edge effects may be created intentionally at least in the middle portion of the electrode so as to bring the current density in the middle portion of the electrode more in line with the enhanced current density caused by the edge effects at the ends. A number of alternative techniques and structures for achieving this basic objective are disclosed below. Although the '382 patent discloses coiled electrodes having spaces between coil windings (e.g., Figs. 7 and 8) that would appear to inherently achieve the goal of introducing edges throughout a coiled electrode, the teaching of the '382 patent is limited to coil electrodes. It does not disclose or suggest introducing additional edges into any other type of electrode structure.

As the gaps between spirals of a coiled electrode become smaller, more electromagnetic coupling occurs between the spirals and the electrode behaves more like a conventional ring electrode insofar as edge effects at the electrodes' ends are concerned. The benefit of introducing additional edges throughout the electrode therefore diminishes as the spirals are brought closer together. It is thus desirable to keep the gaps relatively large so as to maximize that benefit. If the gaps are made too large, however, the electrode may not ablate tissue evenly, i.e., a scalloped, rather than uniform, ablation patter may result. Advantageously, in accordance with an aspect of the invention, the spirals of two or more electrically isolated electrodes may be interleaved with each other so that the width of the gaps between each electrode's spirals can be increased, while still creating a uniform lesion pattern by separately energizing each of the respective electrodes.

The second approach disclosed herein for minimizing the adverse impact of edge effects in an ablation electrode involves somehow causing the portions of the electrode that are subjected to higher current densities due to edge effects to be responsible for ablating more tissue than the other portions of the electrode, thereby ensuring that the tissue along the entire path of an intended lesion pattern is subjected to a substantially uniform density of ablation energy. A number of techniques for achieving this objective also are disclosed, including, for example, separating spirals of a coiled electrode at locations near the ends of the electrode, while not separating, or separating to a lesser extent, the spirals in the inner portion of the coiled electrode.

It should be appreciated that coiled electrodes having uniform spacing between adjacent windings, like those depicted in Figs. 7 and 8 of the '382 patent (discussed above), also suffer from "edge effects" at their ends. Indeed, as noted above, the significance of the edge effect at the ends of such a coiled electrode tends to increase as the spacing between the spirals becomes smaller. Notably, in an effort to maximize the flexibility of its electrodes and facilitate the placement of temperature sensing elements, the '382 patent suggests exactly the opposite approach as that suggested herein. That is, for the electrodes depicted in Figs. 12 and 13 of the '382 patent, it is suggested that the electrode windings be spaced closer together at the electrode's ends than in the middle. This suggested spacing of the windings would tend to exacerbate the edge effect problem of the electrode, rather than ameliorate it, because the portions of the electrode having the highest current densities would also be the portions having the largest number of windings per unit length.

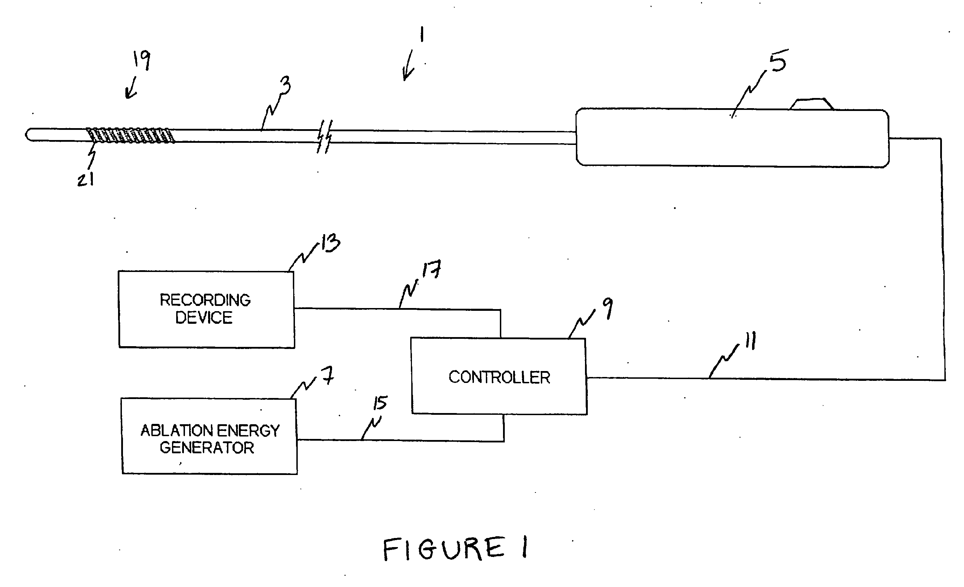

Fig. 1 illustrates an overview of a catheter system that may be used in electrophysiology procedures in accordance with embodiments of present invention. The system may include a catheter 1 having a flexible shaft 3 and a control handle 5. An ablation energy generator 7 may be used for generating ablation energy when the catheter 1 is used in ablation applications, and may transmit ablation energy to the catheter 1 via a controller 9 and a cable 11. A recording device 13 may optionally be included in the catheter system to record signals originating from the catheter 1, e.g., from an electrode or temperature sensor on the catheter.

The controller 9 may, for example, be a QUADRAPULSE RF CONTROLLER™ device available from C.R. Bard, Inc., Murray Hill, New Jersey. As shown, the ablation energy generator 7 may be connected to the controller 9 via a cable 15. The recording device 13 may be connected to the controller 9 via a cable 17. When used in an ablation application, the controller 9 may be used to control ablation energy, provided by the ablation energy generator 7, to the catheter 1. When used in a recording application, the controller 9 may be used to process signals from the catheter 1 and provide these signals to the recording device 13. Although illustrated as separate devices, the ablation energy generator 7, recording device 13, and/or controller 9 may be incorporated into a single device. It should further be appreciated that although both the ablation energy generator 7 and recording device 13 are illustrated in Fig. 1, both of these devices need not be incorporated in the catheter system in accordance with the present invention.

Although the embodiment of Fig. 1 and the description that follows shows and describes the electrode 21 as being mounted on a catheter, it should be appreciated that an electrode configured according to one or more aspects of the invention may alternatively be mounted on any type of shaft or probe, and the invention is not necessarily limited to a catheter-mounted electrode.

As shown, the catheter I of Fig. 1 may include a distal end 19 having an electrode 21. As described below, a number of different constructions are possible for the one or more electrodes that may be included on the distal end 19 of the catheter 1, or other electrode-carrying device.

Fig. 2 illustrates the distal end 19 of the catheter I (Fig. 1) having an electrode 23 shaped in a coil configuration, similar to the electrodes shown, for example, in Figs. 5 and 6 of the '382 patent (discussed above). Like those electrodes, the coil shape of the electrode 23 permits it to be flexed to at least some degree. The electrode 23 may be, for example, between approximately 4 mm and 10 mm in length so as to provide a sufficient surface area for tissue ablation. Flexibility of the electrode 23 may be useful, for example, when the catheter 1 is a steerable catheter, so that the electrode may be flexed as the corresponding portion of the shaft 3 is maneuvered. Flexibility of electrode 23 may also be useful for conforming the electrode to anatomy and for use with catheters that are shaped to or have the capability of conforming to anatomy.

Unlike the electrodes disclosed in the '382 patent, the conductors of the electrode 23 are recessed within the outer circumferential surface of the distal end 19 so as to provide a low or flush profile. A low or flush profile may increase the safety of the catheter by reducing the risk of damage to the tissue caused by the electrode. While the embodiment of Fig. 2 shows an electrode that is fully recessed, i.e., the outer surface of the electrode 23 is substantially flush with the outer surface of the portions of the catheter body that abut the edges 25, it should be appreciated that the electrode may alternatively be recessed only partially within the catheter body so that the windings of the electrode 23 protrude slightly above the outer surface of the body of the catheter 1.

An electrode constructed in the manner shown in Fig. 2 may possess some potentially undesirable electrical properties. For example, when an RF signal is applied to such an electrode, electric fields are generated that are distributed within the electrode in an uneven manner. Typically, the electric fields will be greatest at the boundaries of the electrode and, particularly, at any boundary having a sharp transition. As noted above, this phenomenon is variously referred to as an edge effect, fringing, or charge crowding. In the electrode 23 of Fig. 2, the greatest edge effect occurs at each end 25 of the electrode. A similar edge effect may be observed at the ends of a conventional ring-shaped electrode.

Because an electrode exhibiting edge effects yields an uneven distribution of applied RF energy, charring of tissue or coagulation of blood may result if the electrode is used in an ablation electrophysiology catheter procedure. Further, lesions will tend to develop more quickly in tissue in contact with regions of the electrode having a higher concentration of RF energy, which may limit the ability of the electrode to create deep and/or continuous lesions. An additional challenge exists for electrodes having a longer length, as it becomes more difficult to uniformly deliver energy at all points on the surface of an electrode as the surface area of the electrode increases.

Fig. 3 illustrates the distal end 19 of the catheter 1 having an electrode 29 in accordance with another embodiment of the invention. As shown, the electrode 29 of Fig. 3 is similar to the electrodes shown in Figs. 7 and 8 of the '382 patent in that the coiled electrode comprises a conductor forming a series of spirals 31, where adjacent spirals 31 are separated by gaps 33. As with the embodiment shown in Fig. 2, however, the spirals of an electrode may be at least partially recessed within the catheter body so as to form a flush or low profile.

The inclusion of gaps 33 between the spirals 31 result in edge effects being created at the two edges of each spiral 31, along the entire length of the electrode 29, rather than just at the ends 28 of the electrode 29. Thus, the introduction of these additional edges serves to reduce the significance of the edge effects at the ends 28, thereby causing the distribution of current densities (and resulting electric field strength) to be more uniform along the length of the electrode. In practice, the uniformity in the electric field strength between the ends 28 and the middle 30 of the electrode 29 tends to increase as the width of the gaps 33 increases. The reason this occurs is that, as the gaps become smaller, the electromagnetic coupling (or proximity effect) between the spirals causes the electrode 29 to behave more like a traditional ring electrode in terms of its edge effects, i.e., it exhibits more pronounced edge effects at its ends 28.

Thus, as the distance between spirals 31 increases, the flux linkage between the spirals decreases, and a more uniform current density along the length of the electrode 29 is achieved. However, if the distance between the spirals 31 is too large, the density of spirals per unit length may be insufficient to create a beneficial lesion during ablation, or an undesirable scalloped lesion pattern may be formed. Thus, it is desirable to choose an optimum balance between reduced electromagnetic influence between spirals and efficacy of ablation. This results in more uniform energy delivery and therefore reduces charring and increases the uniformity of lesions. In one example implementation of the electrode 29 of Fig. 3, the gaps 31 are approximately the same width as the spirals 33.

Fig. 4 illustrates an electrode configuration that allows an increased distance between spirals, thereby minimizing the edge effect at the ends of the electrode, while at the same time maintaining a suitable density of spirals per unit length. As shown, the distal end 19 of the catheter 1 may include an ablation electrode 54 comprising a first conductor 57 having spirals 56 interleaved between spirals 58 of a second conductor 59. The first and second conductors 57, 59 of the electrode 54 may be electrically isolated from one anther and independently energized. For example, each of the first and second conductors 57, 59 may be respectively connected to controller 9 (Fig. 1) via first and second wires (not shown), and may receive different signals. In one example, the first and second conductors 57, 59 are energized individually in a pulsed sequential fashion for complete thermal coverage.

It should be appreciated that a number of variations on the described embodiment are possible. For example, an electrode may be provided having three or more conductors with interleaved spirals, and such conductors may be energized in a sequential or even a random fashion. The spirals of the various conductors may also be spaced from one another either in a uniform or non-uniform pattern (e.g., with spaces between spirals being greater at the ends of the electrode than in the middle), rather than being contiguous as shown in the illustrated embodiment, so as to achieve additional advantages discussed herein. Moreover, the conductors forming the spirals may be completely recessed within the body of the catheter 1 so that the upper surface of the conductor/body junction is substantially flat (as illustrated in Fig. 4), may be partially recessed within the body of the catheter 1 so that only a slight protrusion of spirals results, or may simply be disposed on top of the catheter's body without the catheter body being recessed in any fashion.

Fig. 5 illustrates a further embodiment of the invention in which spirals 49 of an electrode 47 are spaced along the length of the electrode 47 so as to minimize the impact of edge effects near the edges 60. In the example embodiment shown, the spirals 49 of electrode 47 are contiguous in the center portion 53 of the electrode, while gaps 51 exist between the spirals 49 in the end portions 55 of the electrode 47. In one implementation, these gaps 51 may be approximately equal in width to the width of the wire of the electrode 47. In the configuration of Fig. 5, although the electrode 47 will exhibit an uneven current distribution when energized, the evenness of the distribution of heat within the tissue along the length of electrode 47 will be improved significantly because the spirals are spaced further apart in the end portions 55 (where the current densities are highest due to edge effects in those regions) than in the middle portion 53.

Alternatively, as illustrated in Fig. 6, gaps 62 between spirals 66 of an electrode 61 may be created so that they are largest near the ends 68 of the electrode 61, and gradually become smaller toward the middle 70 of the electrode 61. Ideally, a spacing can be achieved using this technique by which the greater spacing near the ends 68 accounts exactly for the greater current density in those regions due to edge effects, thereby achieving a substantially uniform thermal profile across the entire length of the electrode 61.

As with the other embodiments described above, it should be appreciated that the conductors forming the spirals in the embodiments of Figs. 5 and 6 may be completely recessed within the body of the catheter 1 so that the upper surface of the conductor/body junction is substantially flat (as illustrated in Fig. 5), may be partially recessed within the body of the catheter I so that only a slight protrusion of spirals results, or may simply be disposed on top of the catheter's body without the catheter body being recessed in any fashion (as illustrated in Fig. 6). In the latter two cases, the protrusion of the electrode may enhance contact between the electrode and the tissue being ablated.

In addition, it should be appreciated that the foregoing technique of spacing the spirals more closely together in the middle portion of an electrode than near the ends may also be employed with a multiple conductor configuration such as that described above in connection with Fig. 4.

Although not illustrated, it should be appreciated that a similar effect to that described above in connection with Figs. 5 and 6 may be achieved using an electrode wire having a non-uniform cross-sectional area. For example, an electrode comprising: (1) a first group of spirals at the center that are formed using wire having a relatively large cross-sectional width, and (2) second groups of spirals at the ends that are formed using wire having a relatively smaller cross-sectional width, may achieve a similar result. Alternatively, the cross-sectional width of the wire may also increase gradually from the ends to the middle of the coiled electrode. In either such embodiment, the spacing of the spirals may be uniform or may form some non-uniform pattern like those discussed above in connection with Figs. 5 and 6 (e.g., with larger spaces near the ends of the electrode than in the middle).

Further, although a temperature sensing capability was not described above in connection with the embodiments of Figs. 2-6, it should be appreciated that such a capability may be important during ablation because, without it, overheated tissue may explode or char, releasing debris into the bloodstream. Fig. 7 illustrates a cross-sectional view of the distal end 19 of the catheter 1 in which a temperature sensor 63 is shown disposed within a recess 64 below a conductor 72 of an electrode. The temperature sensor 63 may be a thermocouple, thermistor, or any other device for sensing temperature. Although the temperature sensor 63 is shown disposed below the electrode 61, the sensor 63 may alternatively be disposed above or adjacent the electrode 61, and may be either in direct or indirect contact with the tissue.

The temperature detected at the distal end 19 may be used to provide feedback for control of the ablation energy generator 7 (Fig. 1). As shown, the temperature sensor 63 may be electrically connected to the controller 9 (Fig. 1) via a wire 65, which transmits temperature information along catheter I and the cable 11, which connects the catheter 1 to the controller 9. The electrode 61 may be electrically connected to the controller 9 via a wire 67, which transmits RF energy along catheter 1 and the cable 11. The wire 67 may connected to the end of the electrode (as shown), or may alternatively be connected to a middle portion, or some other portion, of the electrode.

It should be appreciated that any of the embodiments described herein may include a temperature sensor configured and arranged in a similar manner as the temperature sensor 63 of Fig. 7. Alternatively, some embodiments of the invention may employ other suitable temperature control techniques such as impedance control or power titration of the energy applied to the electrode. Further, the duty cycle of applied energy may be selected to allow cooling of the tissue between energy pulses, or the pulse width of the applied energy may be selected to avoid overheating of the tissue.

Figs. 8 and 9 illustrate the distal end 19 of the catheter 1 having non-coiled electrodes in accordance with further embodiments of the invention.

For example, Fig. 8 illustrates an electrode 71 having alternate ridges 73 and grooves 75. The presence of ridges 73 and grooves 75 creates edge effects along the longitudinal length of the electrode 71, at the junctions between the ridges and grooves. Hence, the edge effects that would normally be present at the ends of a ring-shaped electrode are present in the electrode 71. However, the effects are balanced by the edge effects present towards the center of the electrode 71, which tend to cause a more even distribution of the electric field when the electrode 71 is energized. Although the grooves 75 of the electrode 71 form a spiral, it should be appreciated that a number of alternative groove configurations may be suitable to create the desired effect. For example, circular or linear recesses may be used. Further, although grooves or recesses have been described, holes traversing the thickness of the electrode 71 may instead be formed. The holes, grooves, and recesses described may be formed using a number of techniques including, but not limited to, machining or drilling.

Fig. 9 illustrates an electrode 77 having alternate conductive portions 79 and insulative portions 81 at its surface. The insulative portions 81 may, for example, comprise a dielectric interleaved between the conductive portions 79 in a spiral formation. Alternatively, the insulative portions 81 may comprise a dielectric layer formed on the surface of the electrode 77. As similarly described above in connection with Fig. 8, the presence of conductive portions 79 and insulative portions 81 creates edge effects along the longitudinal length of the electrode 77, at the junctions between the insulative and conductive portions. With such a configuration, the edge effects at the ends 82 of the electrode 77 are balanced by the edge effects introduced towards the center 84 of the electrode 77, thereby causing a more even distribution of the electric field when the electrode 77 is energized. Although the insulative portions 81 form a spiral in the example shown, it should be appreciated that a number of alternative configurations may be suitable to create the desired effect. For example, circular or linear insulative portions 81 may be used. The insulative portions 81 described may be formed using a number of techniques including, but not limited to, masking or laser stripping of insulation.

It should be appreciated that the technique of using some sort of insulative or dielectric material 81 to form the pattern shown in Fig. 9 may similarly be employed to emulate any of the other electrode patterns described herein, and thereby achieve similar results. For example, rather than winding conductors about the catheter 19 in the manner shown in Figs. 3, 5 and 6 above, a dielectric material could instead be drawn or otherwise disposed onto the portions of a conventional ring electrode at the locations corresponding to the gaps between the spirals illustrated in those examples.

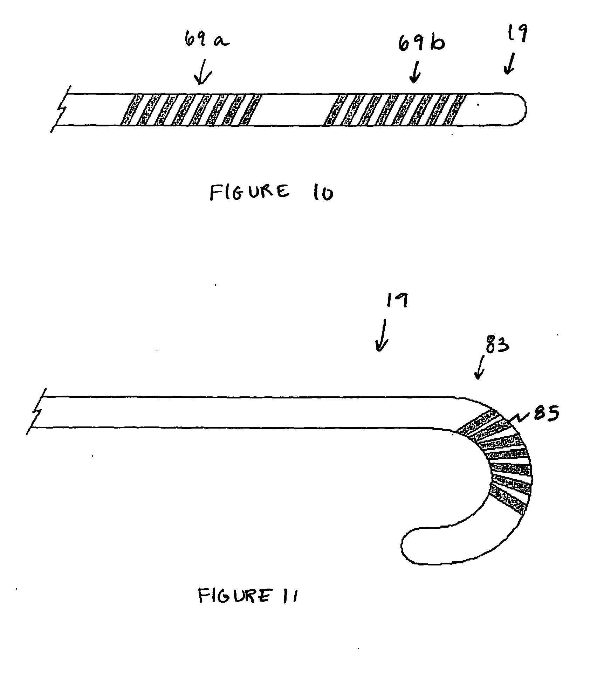

Fig. 10 illustrates the distal end 19 having a pair of electrodes 69a and 69b, which may be configured to create a uniform lesion during ablation. It should be appreciated that two or more electrodes may be included on the distal end 19 for any of the embodiments described herein, and that any combination of described electrodes may be used. Further, any of the electrodes described herein may be movable (e.g., slidable) along the longitudinal length of the catheter, which may assist in the creation of a linear lesion.

In one example, the electrodes 69a and 69b are formed of a material that is an ideal conductor. One exemplary material that is both an ideal conductor and biocompatible is platinum. In accordance with an alternate embodiment of the invention, the electrodes 69a and 69b may be formed of a material that is a non-ideal conductor. A non-ideal conductor may include any material that possesses an electric field gradient within the material when the material is energized. Stainless steel and tungsten are two examples of a material that is both biocompatible and a non-ideal conductor. Non-ideal conductors tend to exhibit more uniform electric field characteristics than ideal conductors. Thus, more even lesion formation may be achieved using a non-ideal conductor. In accordance with a further alternate embodiment of the invention, the electrodes 69a and 69b may be formed of a material that is a semiconductor or metalloid. In one example, a conductive material may be doped to achieve desirable properties. Any of the electrodes described herein may formed of either an ideal conductor, non-ideal conductor, or semiconductor or metalloid material, in accordance with the invention. The particular material selected may be chosen according to the desired application.

Fig. 11 illustrates the distal end 19 having an arcuate curve 83 and an electrode 85 disposed on a portion of the catheter including the arcuate curve 83. The electrode 85 may be configured to create a uniform lesion during ablation. The arcuate curve 83 may be fixed or may be actively controlled via a steering mechanism of the catheter. It should be appreciated that a portion of the distal end 19 may be curved, bent, or steerable in any of the embodiments described herein in accordance with the invention.

It should be appreciated that the above-described embodiments are merely intended to illustrate possible implementations of the present invention, and various modifications and improvements will readily occur to those skilled in the art. Such modifications and improvements are intended to be within the scope of the invention. Accordingly, the foregoing description is by way of example only, and is not intended as limiting.

## Claims

1. An apparatus for ablating tissue, comprising:
a shaft (3); and
a tissue-ablating electrode (29) comprising a first end portion (28) and a middle (30) portion supported by respective lengthwise sections of the shaft, **characterised in that** the total energy-emitting surface area of the electrode per unit length of the shaft is greater for the middle portion of the electrode than for the first end portion of the electrode.

2. The apparatus of claim 1, wherein the first end portion (28) of the electrode comprises a first section of a coiled conductor (31), which first section has spirals that are spaced apart from one another.

3. The apparatus of claim 2, wherein the middle portion (53) of the electrode comprises a second section of the coiled conductor, which second section has spirals (49) that are closer together than the spirals of the first section of the coiled conductor.

4. The apparatus of claim 3, wherein at least two of the spirals in the second section of the coiled conductor touch each other.

5. The apparatus of any of claims 1-4, wherein the electrode comprises a coiled conductor having spaces between at least some of its spirals (47,66) and a cross-sectional width of the conductor forming the spirals is narrower in the first end portion (55,68) than in the middle portion (53,70).

6. The apparatus of any of claims 1-5, wherein the electrode comprises at least two separate coiled conductors (57,59) having interleaved spirals.

7. The apparatus of any of claims 1-6, wherein the electrode comprises a coiled conductor (61) having spaces (62) between adjacent spirals (66) that gradually decrease in size beginning at each end of the electrode and ending in a middle of the electrode.

8. The apparatus of claim 1, wherein the electrode comprises a conductor of a generally cylindrical shape that is partially masked with a non-conductive substance at least in the first end portion of the electrode.

9. The apparatus of claim 1, wherein the electrode comprises a conductor of a generally cylindrical shape.

10. The apparatus of any of claims 1-9, wherein the electrode further comprises a second end portion opposite the first end portion, and wherein the total energy-emitting surface area of the electrode per unit length of the shaft is greater for the middle portion of the electrode than for the second end portion of the electrode.

11. The apparatus of any preceding claim, in combination with an ablation energy generator (7) operatively coupled to the electrode to enable the ablation energy generator to transmit sufficient energy to the electrode to ablate tissue.

12. The apparatus of any preceding claim, wherein the shaft comprises a distal end (19) of an elongated catheter.

13. The apparatus of any preceding claim, wherein the distal end of the elongated catheter is steerable.

14. The apparatus of claim 12, wherein the electrode is mounted on the shaft such that at least a portion of an end of the electrode is disposed at least partially below an annular surface of the shaft that is adjacent the end of the electrode.

15. The apparatus of any preceding claim, wherein the electrode is mounted on the shaft such that at an upper surface of the end of the electrode is substantially flush with the annular surface of the shaft that is adjacent the end of the electrode.

## Patentansprüche

1. Vorrichtung zur Ablation von Gewebe, umfassend:
eine Stange (3); und
eine gewebeabtragende Elektrode (29) mit einem ersten Endbereich (28) und einem mittleren Bereich (30), die jeweils von Längsabschnitten der Stange getragen werden, **dadurch gekennzeichnet, dass** die gesamte energieemittierende Fläche der Elektrode pro Längeneinheit der Stange für den mittleren Bereich der Elektrode größer als für den ersten Endbereich der Elektrode ist.

2. Vorrichtung nach Anspruch 1, bei welcher der erste Endbereich (28) der Elektrode einen ersten Abschnitt eines gewickelten Leiters (31) umfasst, wobei der erste Abschnitt Windungen aufweist, die zueinander getrennt angeordnet sind.

3. Vorrichtung nach Anspruch 2, bei welcher der mittlere Bereich (53) der Elektrode einen zweiten Abschnitt des gewickelten Leiters umfasst, wobei der zweite Abschnitt Windungen (49) aufweist, die zueinander näher sind als die Windungen des ersten Abschnitts des gewickelten Leiters.

4. Vorrichtung nach Anspruch 3, bei welcher wenigstens zwei der Windungen im zweiten Abschnitt des gewickelten Leiters einander berühren.

5. Vorrichtung nach einem der Ansprüche 1 - 4, bei welcher die Elektrode einen gewickelten Leiter umfasst, der Zwischenräume zwischen wenigstens einigen seiner Windungen (47, 66) aufweist, und eine Querschnittsbreite des Leiters, der die Windungen bildet, am ersten Endbereich (55, 68) enger als im mittleren Bereich (53, 70) ist.

6. Vorrichtung nach einem der Ansprüche 1 - 5, bei welcher die Elektrode wenigstens zwei getrennte gewickelte Leiter (57, 59) umfasst, die überlappende Windungen aufweisen.

7. Vorrichtung nach einem der Ansprüche 1 - 6, bei welcher die Elektrode einen gewickelten Leiter (61) umfasst, der Zwischenräume (62) zwischen benachbarten Windungen (66) aufweist, deren Größe von jedem Ende der Elektrode bis zur Mitte der Elektrode schrittweise abnimmt.

8. Vorrichtung nach Anspruch 1, bei welcher die Elektrode einen Leiter von allgemein zylindrischer Form umfasst, der teilweise mit einem nicht leitenden Material wenigstens am ersten Endbereich der Elektrode abgedeckt ist.

9. Vorrichtung nach Anspruch 1, bei welcher die Elektrode einen Leiter von im Allgemeinen zylindrischer Form umfasst.

10. Vorrichtung nach einem der Ansprüche 1 - 9, bei welcher die Elektrode des weiteren einen zweiten Endbereich, entgegengesetzt zum ersten Endbereich, aufweist und wobei die gesamte energieemittierenden Fläche der Elektrode pro Längeneinheit der Stange für den mittleren Bereich der Elektrode größer als für den zweiten Endbereich der Elektrode ist.

11. Vorrichtung nach einem der vorstehenden Ansprüche, die in Kombination mit einem Ablationsenergiegenerator (7) mit der Elektrode operativ gekoppelt ist, um dem Ablationsenergiegenerator zu ermöglichen, ausreichend Energie an die Elektrode zu übertragen, um Gewebe abzutragen.

12. Vorrichtung nach einem der vorstehenden Ansprüche, bei welcher die Stange ein distales Ende (19) eines länglichen Katheters umfasst.

13. Vorrichtung nach einem der vorstehenden Ansprüche, bei welcher das distale Ende des länglichen Katheters verstellbar ist.

14. Vorrichtung nach Anspruch 12, bei welcher die Elektrode auf der Stange so angebracht ist, dass wenigstens ein Bereich eines Endes der Elektrode wenigstens teilweise unterhalb einer ringförmigen Oberfläche der Stange, die in Nähe des Endes der Elektrode ist, angeordnet ist.

15. Vorrichtung nach einem der vorstehenden Ansprüche, bei welcher die Elektrode so auf der Stange angebracht ist, dass eine obere Oberfläche des Endes der Elektrode im Wesentlichen bündig mit der ringförmigen Oberfläche der Stange ist, die in Nähe des Endes der Elektrode ist.

## Revendications

1. Appareil pour l'ablation de tissus, comprenant :
un corps (3) ; et
une électrode d'ablation de tissus (29) comprenant une première partie d'extrémité (28) et une partie centrale (30) supportée par des parties longitudinales respectives du corps, **caractérisé en ce que** la surface totale émettant de l'énergie de l'électrode par unité de longueur du corps est plus grande dans la partie centrale de l'électrode que dans la première partie d'extrémité de l'électrode.

2. Appareil selon la revendication 1, dans lequel la première partie d'extrémité (28) de l'électrode comprend une première section d'un conducteur enroulé (31), laquelle première partie possède des tours espacés les uns des autres.

3. Appareil selon la revendication 2, dans lequel la partie centrale (53) de l'électrode comprend une deuxième section du conducteur enroulé, laquelle deuxième section possède des tours (49) plus rapprochés que les tours de la première section du conducteur enroulé.

4. Appareil selon la revendication 3, dans lequel au moins deux des tours de la deuxième section du conducteur enroulé se touchent.

5. Appareil selon l'une quelconque des revendications 1 à 4, dans lequel l'électrode comprend un conducteur enroulé avec des espaces entre certains au moins de ses tours (47, 66) et une largeur en section du conducteur formant les spirales est plus étroite dans la première partie d'extrémité (55, 68) que dans la partie centrale (53, 70).

6. Appareil selon l'une quelconque des revendications 1 à 5, dans lequel l'électrode comprend au moins deux conducteurs enroulés (57, 59) séparés ayant des tours entremêlés.

7. Appareil selon l'une quelconque des revendications 1 à 6, dans lequel l'électrode comprend un conducteur enroulé (61) ayant des espaces (62) entre des tours (66) adjacents dont la taille diminue graduellement à partir de chaque extrémité de l'électrode et jusqu'au milieu de l'électrode.

8. Appareil selon la revendication 1, dans lequel l'électrode comprend un conducteur d'une forme globalement cylindrique qui est partiellement masqué par une substance non conductrice au moins dans la première partie d'extrémité de l'électrode.

9. Appareil selon la revendication 1, dans lequel l'électrode comprend un conducteur de forme globalement cylindrique.

10. Appareil selon l'une quelconque des revendications 1-9, dans lequel l'électrode comprend en outre une deuxième partie d'extrémité opposée à la première partie d'extrémité, et dans lequel la surface totale émettant de l'énergie de l'électrode par unité de longueur du corps est plus grande dans la partie centrale de l'électrode que dans la deuxième partie d'extrémité de l'électrode.

11. Appareil selon l'une quelconque des revendications précédentes, associé à un générateur d'énergie d'ablation (7) en liaison opérationnelle avec l'électrode pour permettre au générateur d'énergie d'ablation de transmettre suffisamment d'énergie à l'électrode pour réaliser l'ablation de tissus.

12. Appareil selon l'une quelconque des revendications précédentes, dans lequel le corps comprend une extrémité distale (19) d'un cathéter allongé.

13. Appareil selon l'une quelconque des revendications précédentes, dans lequel l'extrémité distale du cathéter allongé est orientable.

14. Appareil selon la revendication 12, dans lequel l'électrode est montée sur le corps de telle façon qu'une partie au moins d'une extrémité de l'électrode soit disposée au moins partiellement en dessous d'une surface annulaire du corps adjacente à l'extrémité de l'électrode.

15. Appareil selon l'une quelconque des revendications précédentes, dans lequel l'électrode est montée sur le corps de telle façon qu'une face supérieure de l'extrémité de l'électrode soit sensiblement de niveau avec la surface annulaire du corps adjacente à l'extrémité de l'électrode.
